# EUROPEAN PATENT SPECIFICATION

(11) **EP 1 744 726 B1**
(45) Date of publication and mention of the grant of the patent: **21.07.2010**
(21) Application number: 05731686.1
(22) Date of filing: 23.03.2005
(51) Int. Cl.: A61K 8/38, A61K 8/21, A61K 33/16

(54) **Use of an emulsion comprising at least one perflouride compound for the treatment of a micro angiopathy of the skin**
Verwendung einer Emulsion enthaltend mindestens eine perfluoride Verbindungen zur Behandlung von einer Mikro-Angiopathie der Haut
Utilisation d'une émulsion comprenant au moins une perfluoride pour la traitement d'un micro-angiopathy de la peau

(30) Priority: 31.03.2004 RU 2004109556
(43) Date of publication of application: 24.01.2007
(73) Proprietor: Otkrytoe Aktsionernoe Obschestvo Faberlic, Moskovskaya obl. 143980 (RU)
(72) Inventor: KLJUSHNIK, Tatiana Pavlovna, Moscow, 129981 (RU); LUSHNIKOV, Konstantin Vasilievich, Chaikovsky, Permskaya obl., 617762 (RU); CHEMERIS, Nikolai Konstantinovich, Puschino, Moskovskaya obl., 142290 (RU); TIKHONOVA, Irina Valerievna, Tula, 300005 (RU); TANKANAG, Arina Vladimirovna, Kabardino-Balkarskaya Republic, 360022 (RU); SHIBAEV, Nikolai Victorovich, Puschino, Moskovskaya obl., 142290 (RU); KORNEEVA, Rimma Valerievna, Moscow, 117454 (RU)
(74) Representative: Becker Kurig Straus
(86) International application number: PCT/RU2005/000132
(87) International publication number: WO 2005/094778

(56) References cited:
- EP-A- 0 390 306
- US-A- 5 330 681
- ANONYMOUS: "What is the oxygen cosmetic?" INTERNET ARTICLE, [Online] 1 June 2001 (2001-06-01), XP002336057 Retrieved from the Internet: URL:http://web.archive.org/web/20010601064 116/http://www.russian-line.ru/en/beauty/a bout.html> [retrieved on 2005-07-14]
- ANONYMOUS: "Rejuvenating cosmetics ( Aquaftem)" INTERNET ARTICLE, [Online] 21 January 2001 (2001-01-21), XP002336058 Retrieved from the Internet: URL:http://web.archive.org/web/20010121230 400/http://www.russian-line.ru/en/products /young.html> [retrieved on 2005-07-14]
- ANONYMOUS: "Basic cosmetics for dry and normal skin care" INTERNET ARTICLE, [Online] 21 January 2001 (2001-01-21), XP002336059 Retrieved from the Internet: URL:http://web.archive.org/web/20010121230 400/http://www.russian-line.ru/en/products /young.html> [retrieved on 2005-07-14]
- DATABASE WPI Section Ch, Week 199601 Derwent Publications Ltd., London, GB; Class B05, AN 1996-008754 XP002336065 & RU 2 033 163 C1 (NIZAR STOCK CO) 20 April 1995 (1995-04-20)
- DATABASE WPI Section Ch, Week 200048 Derwent Publications Ltd., London, GB; Class B07, AN 2000-530701 XP002336066 & RU 2 143 263 C1 (AKHSYANOV U U) 27 December 1999 (1999-12-27)
- DATABASE WPI Section Ch, Week 200012 Derwent Publications Ltd., London, GB; Class D21, AN 2000-135267 XP002336067 & RU 2 119 790 C1 (NIZAR STOCK CO) 10 October 1998 (1998-10-10) cited in the application
- DATABASE WPI Section Ch, Week 199839 Derwent Publications Ltd., London, GB; Class B05, AN 1998-454911 XP002336068 & RU 2 102 972 C1 (NIZAR STOCK CO) 27 January 1998 (1998-01-27) cited in the application

## Description

This invention relates to the art of cosmetology and/or dermatology and concerns the use of pharmaceutical compositions that affect indices of blood microcirculation in a skin.

The skin, being the biggest by place organ of any human beings, provides protection from unfavorable actions of surrounding world. Conditions of skin integuments, outer appearance of the skin and its barrier qualities depend essentially on physiologies mechanisms that should maintain a skin normal functioning.

Metabolic processes in skin tissues have slow down at unfavorable influence on human beings of factors of outer medium and also at an aging process. As a result, the skin loses its protective properties, its functioning is disturbed, its healthy outer appearance is lost.

Skin microcirculation channels are ones of the most important systems of the skin. Provision of tissues by some blood depends on the functional condition of this system and, consequently, skin nutrition, skin outer appearance and skin ability to provide some barrier functions depending on these conditions. Any disturbance of the blood microcirculation plays essential role in development of the organism predisposition to various disturbances at the action of outer medium unfavorable factors. Such conditions characterized by that the skin being healthy by its outer appearance, possess adjustment reserves that are not enough to withstand outer factors. If the load is high enough, or if the load seems small but acts continuously, the skin reacts negatively. Functional conditions of skin cells are deviate unfavorably, and it reflects in its accelerated aging, loosing attractiveness of the skin outer appearance, and can lead to a disease development.

Various cosmetic and dermatology means are intended to normalize disturbed functions of cells of skin tissues, to improve physiological condition of skin integuments and to assist to obtain by the skin of an attractive and healthy appearance. Cosmetic preparations containing perfluorcarbonates are widely used among many other means.

It was discovered in the end of seventies of the last century that perfluorocompounds are excellent solvents of gases, which are vitally necessary for a living organism, namely, oxygen and carbonic acid gas. These results, and also chemical and biological inertia of perfluorocompounds became a basis for production of blood substitutes functioning as a gas "transports - there were "Fluosol-DA" in Japan, "Oxygent" in the USA and "Perftoran" in Russia.

It was discovered also, that the perfluorocompounds are perspective for making preparations of cutaneous application.

It was shown, in particular, that perfluorcarbonate emulsions are effective oxygen vehicles and they represent a perspective class of compounds, which may be used in the skin care means.

Use of perfluorcarbonates as a means stimulating wound healing and/or improving metabolism in integument tissues was patented (see Russian patent No. 2033163 IPC A61K 33/16, 1992).

Emulsion of perfluorocompounds was described, which is intended for the use in cosmetology. It contains 0.2 to 100 (w/v %) of perfluorcarbonates and 30 to 99 (w %) of phosphatidilcholine. The perfluorocompounds and phospholipides are presented in this emulsion in the form of asymmetric lamellar structures (German patent No. 4221255, A61K 7/48, 1994).

The use of the above-described emulsion is patented in German patent No. 4221268, A61 K 9/127, 1994.

An aqueous emulsion of perfluorocompounds is known, which is stabilized by phospholipides and has in its composition treating means of various actions, namely, dermatological preparations, biostatics, immunomodulators, antiviral and antifungal means, heparin, antibiotics, anesthetics of local application, antihistamine and antipsoriatic preparations and others that are presented in an aqueous phase of the emulsion (German patent No. 4221256, A61K9/127, 1994).

An aqueous emulsion of perfluorocompounds was obtained that contains 1 to 8 (w. %) of nonionogenous surface active substance as an emulator (German patent No. 4236607, A61K 7/48, 1994).

Perfluored inino-bis(polyoxialsiles), co-polymers of polyoxiethylene and polyoxipropylene, ethoxyled ether of a sorbent and a fat acid, non-ionic ethoxyled fluorine contained surface active substances and/or ethoxyled polypropyleneglycols can be used as such emulators. Contents of perfluorocompounds in the emulsion can be 0.02 to 100 (w/v %). Perfluoralkenes and cycloalkanes, perfluored amines and ethers of various structure, bis(perfluoralkile)ethylene, and also their mixtures can be used as the perfluorcompounds.

The emulsion can be used for production of corresponding cosmetic and/or dermatological means of various preparative forms, intended for supplying a skin by the oxygen. (German patent No/ 4236607, A61K 7/48, 1994).

The emulsion can be used for production of corresponding cosmetic and/or dermatological means of various preparative forms, intended for supplying a skin by the oxygen. (German patent No/ 4236607, A61 K 7/48, 1994).

Use of stabilizing emulsion of perfluorcarbonates as a means of biological rejuvenation of skin tissues by means of shift of biochemical processes to indices that are typical for a physiologically younger age is known (see Russian patent No. 2119790 IPC A61K 7/00, 1996).

Use of a water emulsion of perfluorcarbonates stabilized by phospholipides, as a means that indicates a hydrating effect in respect of integument tissues is also known. Relation of the phospholipides to perfluorcarbonates was between 1:5 and 2:1 (by mass), and content of perfluorcarbonates in the emulsion is not more than 100 w/v % (see Russian patent No. 2102972 IPC A61K 7/00, 1997).

Nevertheless, neither these sources of information, nor other ones that are known to the applicant, disclose, describe or obviously predict any influence of perfluorcarbonates emulsion to reserves of the skin blood flow and to the systems that regulate the blood flow. Decrease of reliability of these systems makes conditions for microcirculation distortion. Such reduction of a microcirculation control arises under influencing of various stressor factors, as well as in the process of aging.

*The applicant was the first who discovered and investigated the perfluorcarbonate emulsion property to influence the regulatory systems of the skin blood flow, namely, the neurogenous and the endocrine ones.* The possibility of influencing to the systems that regulate the skin blood flow, increasing the reliability of their work and, at the end, eliminating of derangements of blood microcirculation in the skin for increasing its adjustment reserves, is the practical application of this discovery and investigation of these new properties of emulsions of perfluorcarbonates.

Aim of this invention is the use of an emulsion of individual or several perfluoride compounds for the preparation of a pharmaceutical composition for the treatment of a microangiopathy of the skin. Said pharmaceutical composition influences the regulatory systems of the skin blood flow, namely, the neurogenous and the endocrine ones, and essentially increases the skin blood flow reserves and improves conditions of regulatory blood flow systems - neurogenous and endocrine ones, and improves, by such a way, adaptive reactions of the skin integument.

The blood flow in the skin microcirculatory channels is known as unstable: it changes Neuromediators are regulating substances in the first case. Hormones and biologically active substances, including ones that are produced by vessel endothelium, are ones in the second case. It is known, that endothelium controls the vessels tonus through release of vasoconstriction and vasodilatation factors. Both systems are mutually interdepended in some sense, they works in a close contact. A result of their mutual action is influence on vasoconstriction activity of vessels smooth muscles, which determines a size of the vessel lumen and, consequently, an amount of the blood delivered to the tissues. Active impute of aforementioned systems of the skin blood flow regulation determines the stability of the blood microcirculation process in respect of negative influences.

There is, additionally, so-called, passive blood flow regulation. It determines by content of collagene and elastine fibers in the walls of blood flow vessels. Elasticity and flexibility of the vessels walls and, consequently, their ability to withstand loads, depend on content of the said fibers.

The applicant carried out investigations of general blood microcirculation indices, of blood flow reserves, and of indices that characterized with regulatory systems of the skin blood flow, on patients of various age groups having a "problematic" skin, in which processes of vital activity are slowed, and also on patients that were influenced by a stress. (a load test known as an occlusive assay, when the patient's hand is squeezed by a pneumatic sealing ring, was used as a stress model.) A moderate decrease of general microcirculation indices and an essential decrease of indices that characterizes conditions of neurogenous and endocrine regulatory systems of the microcirculation. Essential diminishing of blood flow reserves was noted at continuous action of stress factors. Aforementioned changes of the indices of microcirculatory channels are, possibly, one of the reasons of worsening of conditions of the skin integument at action of inconvenient exogenous and/or endogenous factors, which lead to loosing attractiveness of the skin outer appearance and to its accelerated aging. The applicant showed, in his next investigations, that perfluorcarbonates emulsion is the means, which eliminates disorders of neurogenous, and endocrine regulation systems of capillary blood flow; and it increases essentially reserves of the skin blood flow.

Any known emulsion of individual perfluoride compounds, or an emulsion of several perfluorides compounds, may be used as the perfluorcarbonates emulsion. For the purposes of this invention, the stabilized perfluorcarbonates emulsion may be introduced in a compound of any preparatory forms such as creams, lotions, ointments, gels and so on. Stabilizing emulsions means, here and below, any physical-chemical disperse systems "liquid in liquid" containing an Any known emulsion of individual perfluoride compounds, or an emulsion of several perfluorides compounds, may be used as the perfluorcarbonates emulsion. For the purposes of this invention, the stabilized perfluoride compounds emulsion may be introduced in a compound of any preparatory forms such as creams, lotions, ointments, gels and so on. Stabilizing emulsions means, here and below, any physical-chemical disperse systems "liquid in liquid" containing an emulator and, if necessary, emulsion stabilizer. Normally, the emulator acts as the stabilizing agent too.

Emulsion content in a ready for use preparative form may be, preferably, from 0.1 to 50 % by weight, or it may be out of these limits.

Perfluoride compounds content in the emulsion lies normally in limits between 10.0 and 50.0 % by volume; these limits are also not limiting.

Pharmaceutical compositions that are prepared on the base of the stabilized perfluoride compounds emulsion may contain, and may not contain, any additional biologically active substances. Such substances as antiseptics, antioxidants, stabilizers, colorants, aromatizes and so on can be introduced into the containment of such means.

Known methods may be used to include the perfluoride compounds emulsion into a pharmaceutical composition. The emulsion may be prepared separately and then it may be introduced into the preparation's composition; or it may be generated in the process of making the given means immediately. To prepare the perfluoride compounds emulsion itself, the water, perfluoride compounds and emulator are mixed in any order and then the mixture is made homogenized.

The base of the pharmaceutical composition may be equally hydrophobias bases that are preferable for pharmaceutical means containing hard particles dispersion, and hydrofoils bases that are most suitable for the preparation of pharmaceutical means containing diluted active components. Emulsion bases of various kinds may be used successfully.

The applicant has used in his investigations various perfluoride compounds emulsions and, in particular, a preparation that is 20 % Aquaftem emulsion on the water-oil base.

Aquaftem is a trade name for perfluoride compounds emulsion that is produced on industrial scale and contains: perfluordecaline 51.0 %, perfluorpolymetylisopropil ester 9.0 %, Poloxamer-188 4.0 %, a conservator (the mixture of diasodidinilurea, metylparabene, propylparabene and propilenglycole) and the water up to 100 %.

Participants of the investigation were five healthy women at the age between 30 and 52 years old. mathematic apparatus allows to segregate and to determine in quantities the functional condition of each regulatory system; it allows, in the processes of load tests, to evaluate adaptive reserves of the skin blood flow.

The following parameters of microcirculation were taken into account in these investigations:
IM - Microcirculation index that reflects a general blood flow level in the microcirculatory channels;
A(E) - endothelial rhythm that reflects a participation of the endothelium in blood flow regulation (metabolism in the endothelium of biologically active substances influencing the microcirculation);
A(N) - neurogenous rhythm that reflects a participation of a peripheral nerve system in blood flow regulation;
A(M) - miogenous rhythm, an input of smooth muscle cells of the walls of resistive vessels and pre capillary sphincters, it is connected with the regulation of blood pressure (it determines vessels lumen).
A(R) - respiratory rhythm that reflects a tonus of venule of the skin microcirculatory channels (indices of a blood outflow that coincide with a breath rhythm - they reflect the pump function of the lungs);
A(C) - cardio rhythm that reflects a tonus of skin arterioles of microcirculatory channels (indices of a blood inflow that coincide with arterioles filing at the blood pumping by the heart);
CBR% - capillary blood flow reserves that reflect adaptive reserves of the skin microcirculatory channels.

Evaluation procedure of the skin blood flow parameters included the measurement of the background microcirculation and the measurement of the microcirculation after 30 minutes of overlaying with the preparation. Procedure of measurement was carried out after twenty-four hours during 3 weeks. The load test (an occlusive assay) was used as the model of the stress factor. This test was carried out during the first three days of the experimentation before overlaying with the preparation, and during the last two days of the measurements, and after overlaying with the preparation during all the experiment.

At the occlusive assay the patient's hand was bound by a pneumatic cuff over the elbow (pressing the artery) for 3 minutes, for to stop the blood flow (occlusion). Then the pressure was removed and a dynamics of normal blood flow reestablishing was determined, by testing reserve functions of microcirculatory channels.

Positive results were obtained at the evaluation of influence of the preparation on the background microcirculation. The results allow to conclude about growing impute of the regulatory systems to maintenance of the normal blood flow.

The indices of *flagsomocies* A(E) and A(N) that reflect an impute of the endothelial and neurogenous components of the blood flow regulation system increase essentially at the action of the preparation.

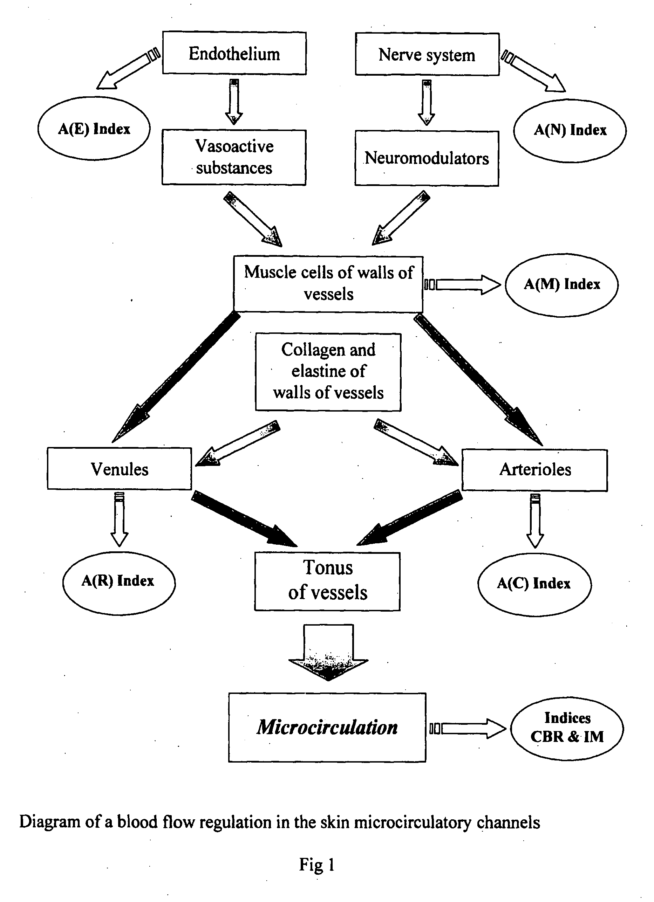

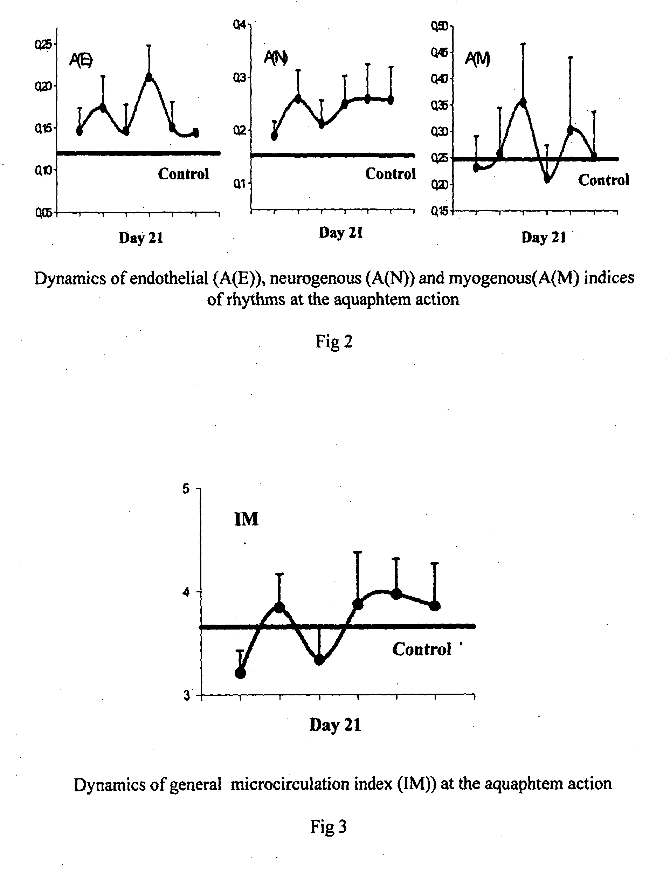

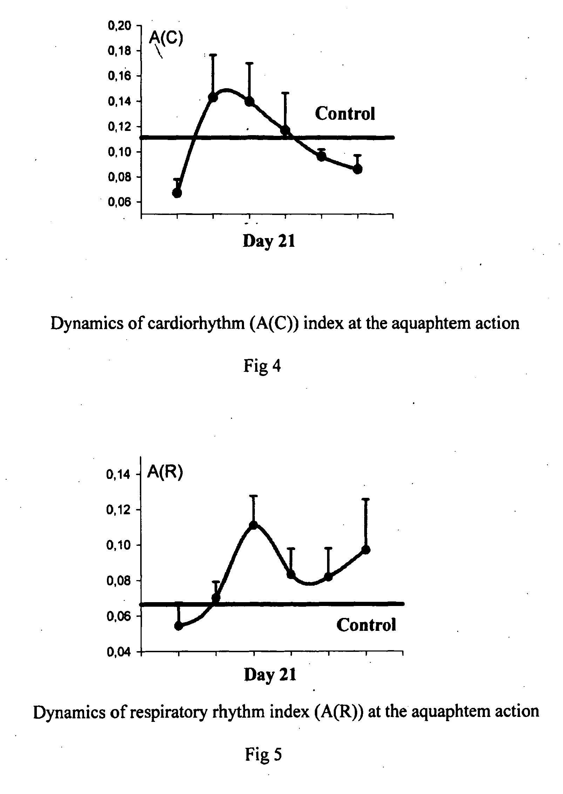

The most expressed and stable effect was obtained at evaluation of the degree of participation of the nervous system in the regulation of the blood flow. Amplitude of *flagsomocies* that reflects a neurogenous control over the microcirculation, was increased essentially as from the first days of the preparation application and it was not changed during all time of the experimentation (Fig. 2). The effect was about 50 %.

The impute of endothelial system into blood flow regulation was also increased as from the first days of the application, it changed by undulating manner (Fig 2). This index was higher, as a whole, up to 20 %, to the end of the experimentation, comparing to the control.

The A(M) index that reflects an impute of the smooth muscle cells of vessels walls into the blood flow regulation had changed by undulating manner and at the end of experimentation (after three weeks of the preparations application) it had no distinctions from the control (Fig. 2). Conclusion was made from this fact, that there were occasional unstable oscillations and that the preparation has no influence on the passive regulation.

The IM of the general microcirculation had changed in a small extent but stable under the preparation influence; it was higher than the control by 6.6 % at the end of the experimentation (Fig. 3).

So, it may be concluded that the preparation action increases the impute of the systems regulating the microcirculation (the neurogenous and endothelial controls), which leads to increase the blood flow reserves and, consequently, to increase of the skin stability with respect of negative influences and, besides, it has favorable effect on the microcirculation itself, by a moderate increase its general level.

Study of preparation influence on a vessels tonus shows results demonstrating changes of physiological conditions of arteriole and venule chains of the microcirculatory channels at the preparation action. The physiological conditions of arterioles and venules depends on mechanisms of active regulation, as well as on factors of passive regulation (contents of elastinous and collagenous fibers in the vessel wall). It was shown that the preparation action on the aforementioned indices is positive also.

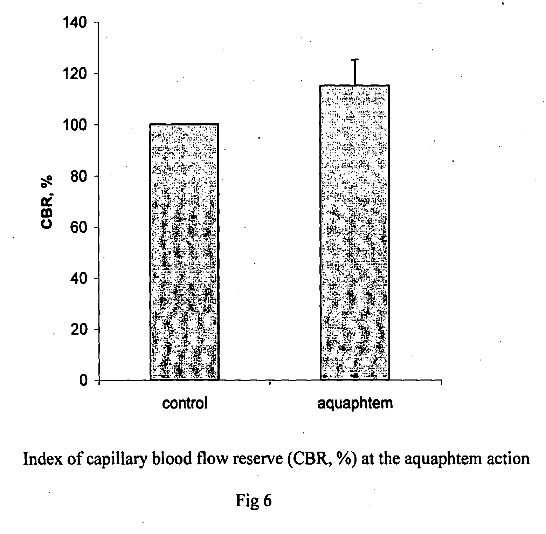

The amplitude of *flagsomocies* in the cardio rhythm diapason A(C) that reflects changes of the diameter of the arterial vessels induced by a pulsation at forcing the blood by the heart was decreased to the end of the experimentation. These data allows to conclude about increase of flexibility of the walls of precapillary arterioles under the preparation influence; it is a favorable sign.( See Fig. 6.)

The amplitude of *flagsomocies* in the breath rhythm diapason A(R) was increasing to the end of the experimentation; it reflects some increase of the venous vessels elasticity and, consequently, it leads to diminishing a risk of arising of venous congestion in the skin. So, the stable growth of sensitivity of post capillary venules to a pumping function of the breath process was observed at application of the preparation (Fig. 7).

The applicant's investigations show that the increase of blood flow active control at the preparation action demonstrates distinctly in the first days of its application already. Nevertheless, the preparation shows long time effects too, which are increase of passive factors of the blood flow regulation, namely, the increase of synthesis of the collagenous and elastine fibers in the vessels walls, because it was shown that the effect of decrease of the amplitude of the cardio rhythm *flagsomocies* was observed after of the expiration of three weeks of the preparation application.

The investigations allow to conclude that the preparation shows the expressed positive influence on the microcirculation indices. The essential increase occurs of the activity of the microcirculation regulation systems and the moderate increase occurs of the general microcirculation. The improve of the conditions of the vessels walls occurs also in arteriolar, as well as in venule chains of the microcirculatory channels.

Investigations in the load test conditions were carried out for evaluation of adaptive reserves of the skin blood flow under the preparation influence. Occlusive assay was used as the load test. The short term and the long term effects were estimated. It was shown that the index of reserve of capillary blood flow (CBR%) was stably increased in average for 15.3 % after 15 minutes of the preparation application (Fig. 8). The preparation action was indicated during all the experimentation.

So, the applicant's investigations showed that the perfluoride compounds emulsions influences positively to the conditions of the blood flow regulatory systems - to neurogenous and endocrine ones; they improve them and, consequently, improve the adaptive reactions of the skin blood flow, and also essentially increase its reserves.

The pharmaceutical compositions containing the perfluoride compounds emulsions can be used in medicine at a micro angiophaties of various etiologies, for example, ones that develop at diabetes, cardio-vascular diseases, psoriases and so on.

Following Examples of implementation of the invention shall not be considered as any limitations of its scope.

### EXAMPLE 1 (not according to the present invention)

### Production of a regenerating cream for a face.

Water (20 kg, 65-70 °C) is poured into a reactor (vacuum 0.4-0.5 atmosphere). A preliminary prepared oil phase in amount of 62.1 kg (the oil phase preparation process will be described below) is added into the reactor. This mixture is homogenized for 5 minutes at 2,000 r/min; 320 kg of water at 65-67 °C is added to the mixture that is homogenized again for 5 minutes at 3,000 r/min. Then 1 kg of carbopol (the carbopol preparation process will be described below) mixed with 50 kg of water, 4.5 kg of Hermabene II and 13.5 kg of glycerin is added to the emulsion and the homogenization is continued at 1,500 r/min. The mixture is cooled up to 34-40 °C and 1 kg of triethanolamine is added to the mixture. Then, at a stirrer being on, aromatizes and 2.5 kg of Aquaphten are added. The resulting substance is mixed up to obtain of the homogeneous mass; pH is measured and is adapted to 6.5-7.0, if necessary, by triethanolamine.

### Production of the oil phase.

9 kg caprilic/capric triglicerides, 6.75 kg of almond oil, 4.5 kg of cocoa oil, 9 kg macadamie oil, 2.25 kg dimeticone, 5.85 kg stearin acid, 11.25 kg emulsion wax, 6.75 kg glyceril stearate and 4.5 kg PEG stearate are filled up with a melting copper. This mixture is heated up to 65-70 °C at continuous agitation. 2.25 kg of tocopherol acetate is added to the oil phase before filing up with the reactor.

### Production of carbopol.

The regenerating cream for a face is produced, which may be used as a prophylactic means against aging. The cream permanent use improves the skin state, its outer appearance because of normalizing exchange processes and reestablishing of microcirculation. It reestablishes the lipide and aqueous balance of the skin and its barrier functions and decreases a transepidermal loss of moisture.

### EXAMPLE II

### Production of a cream for a body.

Water (300 kg, 65-70 °C) is poured into a reactor (vacuum 0.4-0.5 atmosphere). 0.18 kg of EDTA and a preliminary prepared solution of xanthane gum in glycerin (see below). The mixture is homogenized at 2,000 r/min; afterwards 44.5 kg of an oil phase (about production of the oil phase see below) is added and, at the continuous homogenization, the mixture is cooled up to 40 °C. Then, 4.5 kg of hermabene, aromatizes and 9 kg of Aquaphten are added. The resulting substance is mixed up to obtain of the homogeneous mass; pH is measured and is adapted to 6.5-7.0, if necessary, by triethanolamine.

### Production of solution of the xanthane copper.

0.45 kg of the xanthane copper dispersed in 13.5 kg of glycerin is gradually added into water (20 kg, 60 °C) and intensively mixed on a high-speed mixer during 15-20 minutes.

### Production of the oil phase.

9 kg caprilic/capric triglicerides, 6 kg of almond oil, 3.15 kg of cocoa oil, 4.5 kg of Shi oil, 6.75 kg of microcrystalline wax, 9 kg PEG-8 bee wax and 6 kg of ceiled alcohol are filled up with a melting copper. This mixture is heated up to 65-70 °C and agitated up to a uniform mass.

A body cream is obtained for a care of a skin with features of atopic dermatitis, neurodemits and psoriases. This cream use improves a local microcirculation, promotes reestablishment of a lipide barrier and skin cells oxygenation. It improves tropics and reestablishes processes of exchange in tissues.

## Claims

1. Use of an emulsion of individual or several perfluoride compounds for the preparation of a pharmaceutical composition for the treatment of a micro angiopathy of the skin.

2. The use according to claim 1, wherein the micro angiopathy develops at diabetes, cardio-vascular diseases and psoriasis.

## Patentansprüche

1. Verwendung einer Emulsion einzelner oder mehrerer Perfluoridverbindungen zur Herstellung einer pharmazeutischen Zusammensetzung zur Behandlung einer Mikroangiopathie der Haut.

2. Verwendung nach Anspruch 1, wobei die Mikroangiopathie bei Diabetes, Herz-Kreislauferkrankungen und Psoriasis entwickelt wird.

## Revendications

1. Utilisation d'une émulsion d'un ou de plusieurs composés perfluorés pour la préparation d'une composition pharmaceutique pour le traitement d'une micro angiopathie de la peau.

2. L'utilisation selon la revendication 1 dans laquelle la micro angiopathie se développe lors de diabètes, de maladies cardiovasculaires et de psoriasis.
